# FASCICULE DE BREVET EUROPEEN

(11) **EP 3 892 325 B1**
(45) Date de publication et mention de la délivrance du brevet: **16.03.2022**
(21) Numéro de dépôt: 20315142.8
(22) Date de dépôt: 09.04.2020
(51) Int. Cl.: A61N 1/378, A61N 1/372, A61N 1/375, H02N 2/18, H01L 41/113, A61N 1/05, A61N 1/39

(54) **IMPLANT CARDIAQUE AUTONOME DE TYPE CAPSULE LEADLESS, COMPRENANT UN RÉCUPÉRATEUR D'ÉNERGIE À LAME PIÉZOÉLECTRIQUE**
AUTONOMES HERZIMPLANTAT VOM TYP LEADLESS-KARDIOKAPSEL, DAS EIN ENERGIERÜCKGEWINNUNGSSYSTEM MIT PIEZOELEKTRISCHEM PLÄTTCHEN UMFASST
LEADLESS CAPSULE TYPE AUTONOMOUS CARDIAC IMPLANT, COMPRISING AN ENERGY RECOVERY DEVICE WITH PIEZOELECTRIC BLADE

(43) Date de publication de la demande: 13.10.2021
(73) Titulaire: Cairdac, 92160 Antony (FR)
(72) Inventeur: Regnier, Willy, 91160 Longjumeau (FR); Nguyen-Dinh, An, 37520 La Riche (FR)
(74) Mandataire: Dupuis-Latour, Dominique

(56) Documents cités:
- WO-A1-2018/035542
- WO-A1-2019/008431
- CN-A- 109 980 992
- CN-B- 108 540 013
- US-A1- 2018 185 638
- US-A1- 2019 381 325

## Description

### Domaine de l'invention

L'invention concerne les récupérateurs d'énergie, également dénommés *harvesters* ou *scavengers,* qui recueillent l'énergie mécanique résultant de mouvements divers qu'ils subissent, et convertissent cette énergie mécanique en énergie électrique.

Elle concerne plus spécifiquement les récupérateurs de type dit "PEH" *(Piezoelectric Energy Harvester),* qui utilisent comme transducteur mécano-électrique une lame piézoélectrique oscillante couplée à une masse mobile inertielle.

L'invention sera décrite plus particulièrement dans une application de ces récupérateurs d'énergie à des dispositifs médicaux autonomes, notamment les dispositifs de type capsule implantable autonome, en particulier ceux de ces dispositifs qui sont destinés à être implantés dans une cavité cardiaque.

Cette application, si elle est particulièrement avantageuse, ne doit toutefois pas être considérée comme limitative de l'invention, dont les enseignements peuvent être appliqués à de nombreux autres types de dispositifs autonomes incorporant un récupérateur d'énergie de type PEH, que ces dispositifs soient implantables ou non, qu'il s'agisse de dispositifs médicaux ou non.

### Description de la technique antérieure

Dans le domaine des implants médicaux, les récents progrès en matière de miniaturisation de dispositifs actifs et les progrès des sciences du vivant permettent dorénavant le développement d'une grande variété de systèmes miniaturisés implantables totalement autonomes, à des fins de surveillance, de diagnostic ou de traitement. Ces dispositifs mettent en oeuvre des procédures d'implantation moins invasives, plus de confort, des performances accrues et souvent ouvrent l'accès à des nouveaux types de diagnostics et traitements.

Lorsqu'elle est appliquée au domaine des implants médicaux, l'invention concerne plus précisément ceux de ces implants qui incorporent un système d'autoalimentation comprenant un récupérateur d'énergie mécanique associé à un organe de stockage d'énergie intégré tel qu'une batterie rechargeable ou une capacité à hautes performances.

En effet, l'un des aspects critiques de ces dispositifs miniaturisés est celui de l'autonomie électrique. La durée de vie d'un tel implant étant d'environ 8 à 10 ans, compte tenu des très faibles dimensions il n'est pas possible d'utiliser une batterie conventionnelle, même à forte densité.

Le récupérateur d'énergie pallie cet inconvénient en recueillant l'énergie mécanique résultant des mouvements divers subis par le corps du dispositif implanté. Ces mouvements peuvent avoir pour origine un certain nombre de phénomènes se produisant par exemple au rythme des battements cardiaques tels que les secousses périodiques de la paroi sur laquelle est ancré l'implant, les vibrations des tissus cardiaques liées entre autres aux fermetures et ouvertures des valves cardiaques, ou encore les variations de débit du flux sanguin dans le milieu environnant, qui sollicitent l'implant et le font osciller au rythme des variations de débit. L'énergie mécanique recueillie par le récupérateur est convertie en énergie électrique (tension ou courant) au moyen d'un transducteur mécanoélectrique approprié, pour assurer l'alimentation des divers circuits et capteurs du dispositif et la recharge de l'organe de stockage d'énergie. Ce système d'alimentation permet au dispositif de fonctionner en parfaite autonomie électrique pendant toute sa durée de vie.

Cette technique de récupération d'énergie est particulièrement bien adaptée à l'alimentation des capsules autonomes implantées dépourvues de toute liaison physique à un dispositif distant. Ces capsules sont dénommées pour cette raison "capsules leadless", pour les distinguer des électrodes ou capteurs disposés à l'extrémité distale d'une sonde (lead) parcourue sur toute sa longueur par un ou plusieurs conducteurs reliés à un générateur connecté à l'extrémité opposée, proximale.

L'invention n'est toutefois pas limitée à un type particulier de capsule, ni même d'implant, et elle est applicable indifféremment à de nombreux autres types de dispositifs, quelle que soit leur destination fonctionnelle, cardiaque ou autre, médicale ou non.

Dans ce cas d'une application cardiaque, la capsule leadless surveille en continu le rythme du patient et délivre si nécessaire au coeur des impulsions électriques de stimulation, de resynchronisation et/ou de défibrillation en cas de troubles du rythme détecté par la capsule. La capsule leadless peut être une capsule épicardique, fixée à la paroi extérieure du coeur, ou bien une capsule endocavitaire, fixée à la paroi intérieure d'une cavité ventriculaire ou auriculaire, ou bien encore une capsule fixée sur une paroi d'un vaisseau à proximité du myocarde. La fixation de la capsule au site d'implantation est assurée par un système d'ancrage saillant prolongeant le corps de la capsule et destinée à pénétrer dans le tissu cardiaque, notamment au moyen d'une vis.

La capsule comprend par ailleurs divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc. Le WO 2019/001829 A1 (Cairdac) décrit un exemple d'une telle capsule leadless intracardiaque.

Il existe plusieurs types de récupérateurs d'énergie, basés sur des principes physiques différents : système de type mouvement de montre à remontage automatique, système à aimants mobiles, système à soufflet ou analogue récupérant les variations de pression du milieu sanguin, etc. L'invention concerne plus précisément les capsules ou dispositifs implantables analogues dont le récupérateur d'énergie utilise un ensemble pendulaire inertiel soumis aux sollicitations externes décrites plus haut.

Un ensemble pendulaire inertiel met en oeuvre un transducteur comportant dans la capsule une masse mobile, dite "masse sismique" ou "masse inertielle", qui est entrainée au gré des mouvements de la capsule soumise en permanence aux diverses sollicitations externes décrites plus haut. Après chacune de ces sollicitations, la masse inertielle, qui est couplée à un élément élastiquement déformable, oscille à une fréquence propre d'oscillation libre.

L'énergie mécanique de l'oscillation est convertie en énergie électrique par un transducteur mécanoélectrique produisant un signal électrique. Ce signal est délivré à un circuit de gestion de l'alimentation de la capsule, qui redresse et régule le signal électrique pour délivrer en sortie une tension ou un courant continus stabilisés permettant d'assurer l'alimentation des divers circuits électroniques et capteurs de la capsule, ainsi que la recharge de l'organe de stockage d'énergie.

Le transducteur mécanoélectrique peut en particulier être un composant piézoélectrique sollicité de façon cyclique et alternative en flexion de manière à engendrer au sein du matériau qui le constitue des charges électriques qui sont récupérées en surface du composant pour être utilisées par le système d'autoalimentation de la capsule leadless. Ce composant piézoélectrique peut notamment être une lame piézoélectrique encastrée à l'une de ses extrémités et couplée à la masse inertielle à son autre extrémité, qui est libre.

Un tel récupérateur d'énergie de type PEH destiné à l'alimentation d'un implant à partir des oscillations d'une lame piézoélectrique est notamment décrit dans le US 3,456,134 A (Ko) et dans le WO 2019/001829 A1 précité.

On notera que le terme de "lame" doit être entendu dans son sens le plus large, à savoir une bande mince et plate de forme allongée, étant entendu que la forme de cette bande n'est pas nécessairement rectangulaire ni son épaisseur constante (comme dans la description du mode de réalisation particulier qui sera donnée plus bas). Le terme de "lame" au sens de la présente invention recouvre ainsi des éléments pouvant présenter une largeur et/ou une épaisseur qui ne sont pas constantes en direction longitudinale, ainsi qu'éventuellement une déformabilité pouvant aller au-delà d'un unique degré de liberté en flexion.

De nombreuses difficultés techniques doivent toutefois être résolues pour arriver à réaliser des PEH qui soient malgré une miniaturisation extrême à la fois extrêmement performants et parfaitement fiables, avec typiquement une durée de vie sans défaillance ni perte de rendement d'au moins dix ans.

L'expérience montre en particulier que l'une de ces difficultés se présente de façon critique au niveau de l'encastrement de la lame transductrice, là où est maximale la contrainte de la lame dans son mouvement de flexion. Or, c'est précisément cette région, où la courbure de la lame fléchie est la plus importante, qui génère le maximum de charges et donc le maximum d'énergie électrique instantanée délivrée par le PEH.

La manière dont la lame est encastrée dans la pièce qui la supporte et la relie au corps de la capsule est de ce fait un aspect particulièrement délicat de la conception et de la réalisation d'un PEH, et par voie de conséquence d'un implant leadless performant et fiable.

En particulier, il est important que la pression exercée sur le matériau de la lame à l'endroit où cette dernière est pincée dans son encastrement soit répartie de la façon la plus uniforme possible, sans qu'elle soit pour autant :
ni trop faible, car ce serait au détriment i) d'un bon encastrement mécanique permettant de générer les contraintes de flexion recherchées, et ii) d'un bon contact électrique avec les électrodes de surface de la lame (la liaison électrique ne pouvant se faire qu'au niveau de l'encastrement, qui est fixe) ;
ni trop élevée, car une contrainte excessive serait au détriment de la fiabilité.

Les solutions techniques impliquant des collages sont par ailleurs à éviter, car ils sont sur le long terme sujets à des phénomènes d'oxydation et de vieillissement, qui permettent difficilement de garantir la fiabilité absolue requise pour ce genre de dispositifs implantés.

Un autre aspect important dont il faut tenir compte est le centrage précis de la lame par la pièce qui la supporte, au moment de l'assemblage des différents éléments du PEH.

Le WO 2018/122244 A1 (Regnier) décrit en détail divers agencements tentant de répondre à ces multiples contraintes, tels que :
le serrage de la lame entre deux mors soumis à des efforts radiaux contrôlés grâce à un anneau élastiquement déformable, notamment un anneau ovalisé placé autour de ces mors ;
un surmoulage de l'extrémité encastrée de la lame préalablement à son montage, avec éventuellement inclusion d'éléments rigides de renfort ; ou
le pincement de la lame entre deux mors recevant un effort de serrage exercé lors du montage par une pince, puis le surmoulage d'une bague autour de ces mors de façon à assurer la retenue définitive de la lame après suppression de l'effort de la pince.

Il s'agit toutefois de solutions complexes à industrialiser sur le plan de la fabrication mécanique et des tolérances dimensionnelles des différentes pièces, donc de solutions coûteuses à mettre en oeuvre. De plus, les pièces en matière plastique ou les surmoulages restent très sensibles au vieillissement du matériau sur le très long terme, donc ne permettent pas de garantir de façon absolue la fiabilité requise pour un dispositif implanté dans l'organisme.

La présente invention a pour but de résoudre l'ensemble de ces difficultés, en proposant une structure d'encastrement de lame pour PEH qui soit simple à fabriquer et à assembler, et qui pour autant garantisse un très haut niveau de précision de la pression de compression de la lame, avec une excellente fiabilité sur le très long terme, notamment en évitant le recours à des pièces en matière plastique et/ou à des collages.

### RÉSUMÉ DE L'INVENTION

À cet effet, l'invention propose un dispositif médical de type capsule autonome implantable, comprenant, de manière en elle-même connue d'après le WO 2018/122244 A1 précité, un module de récupération d'énergie comprenant un ensemble pendulaire oscillant soumis à des sollicitations externes appliquées au dispositif. L'ensemble pendulaire oscillant comprend une lame piézoélectrique élastiquement déformable, avec une extrémité encastrée et une extrémité opposée libre couplée à une masse inertielle, la lame piézoélectrique étant apte à convertir en un signal électrique oscillant l'énergie mécanique produite par les oscillations de l'ensemble pendulaire. L'enveloppe tubulaire du dispositif loge également une monture de lame, la monture de lame comprenant deux éléments d'appui entre lesquels est prise en sandwich l'extrémité encastrée de la lame piézoélectrique, et un moyen de serrage mutuel des éléments d'appui.

Chaque élément d'appui de la monture de lame comprend i) une pièce intercalaire dont une face interne sollicite en appui une face correspondante de la lame piézoélectrique, et ii) une plaque de pression dont une face interne sollicite en appui une face externe de la pièce intercalaire. De plus, les pièces intercalaires et les plaques de pression des deux éléments d'appui sont traversées de part en part par au moins un alésage commun s'étendant dans une direction transversale par rapport à un axe longitudinal de la lame piézoélectrique, ledit au moins un alésage commun recevant une goupille d'arrêt. Enfin, la monture de lame comprend un moyen de maintien en position et d'assujettissement de la goupille dans ledit au moins un alésage commun.

Selon diverses caractéristiques subsidiaires avantageuses :
le moyen de maintien en position et d'immobilisation de la goupille comprend un soudage laser d'une extrémité libre de la goupille à un bord débouchant de l'alésage commun ;
la pièce intercalaire dont une face interne est en appui contre une face correspondante de la lame piézoélectrique est en contact mécanique avec ladite face correspondante de la lame piézoélectrique. Avantageusement, cette pièce intercalaire comprend en outre sur sa face interne un matériau conducteur apte à établir en outre un contact électrique avec ladite face correspondante de la lame piézoélectrique, au niveau d'une électrode de surface de ladite lame piézoélectrique ;
le dispositif comprend en outre un circuit imprimé interposé entre la pièce intercalaire et la plaque de pression d'au moins l'un des éléments d'appui de la monture de lame, et la plaque de pression comprend, sur une face interne, un matériau conducteur en contact électrique avec une face externe correspondante du circuit imprimé ;
dans ce dernier cas, la pièce intercalaire peut comprendre sur sa face interne un matériau conducteur apte à établir un contact électrique avec ladite face correspondante de la lame piézoélectrique, au niveau d'une électrode de surface de ladite lame piézoélectrique, et
comprendre sur sa face externe un matériau conducteur apte à établir un contact électrique avec une face interne du circuit imprimé, les matériaux conducteurs des faces interne et externe de la pièce intercalaire étant électriquement couplés entre eux. La pièce intercalaire peut en particulier être une pièce monobloc formée d'un seul matériau conducteur ;
les pièces intercalaires, les plaques de pression et la goupille sont chacune des pièces métalliques massives ;
la monture de lame est essentiellement dépourvue d'élément interposé entre les pièces intercalaires et l'extrémité encastrée de la lame piézoélectrique, et/ou essentiellement dépourvue de points de collage ;
l'enveloppe tubulaire allongée loge également une pièce support de forme externe tubulaire conjuguée de la forme interne de l'enveloppe tubulaire, et comprenant à l'une de ses extrémités un réceptacle apte à recevoir et à centrer la monture de lame et à assujettir celle-ci par ajustement sans jeu au niveau de faces externes des plaques de pression ; et/ou
la goupille est une pièce métallique revêtue d'un matériau électriquement isolant apte à éviter une liaison galvanique et/ou un courant de fuite au sein de la lame piézoélectrique ;

La présente invention a également pour objet un procédé de fabrication d'un dispositif tel qu'exposé ci-dessus, le procédé, comprenant les étapes suivantes :
a) obtention d'une lame piézoélectrique ;
b) placement de deux éléments d'appui de part et d'autre d'une extrémité encastrée de la lame piézoélectrique, chaque élément d'appui comprenant i) une pièce intercalaire dont une face interne sollicite en appui une face correspondante de la lame piézoélectrique, et ii) une plaque de pression dont une face interne sollicite en appui une face externe de la pièce intercalaire, avec le cas échéant un circuit imprimé interposé entre la pièce intercalaire et la plaque de pression d'au moins l'un des éléments d'appui de la monture de lame ;
c) mise en place d'une goupille dans un alésage commun traversant de part en part les pièces intercalaires et les plaques de pression des deux éléments d'appui dans une direction transversale par rapport à un axe longitudinal de la lame piézoélectrique ;
d) mise sous pression contrôlée de la lame entre les deux éléments d'appui, jusqu'à atteindre un effort transversal prédéterminé de serrage de la lame piézoélectrique entre les deux éléments d'appui ; et
e) tout en maintenant ledit effort transversal prédéterminé, assujettissement définitif de la goupille dans ledit au moins un alésage commun.

Selon diverses mises en oeuvre avantageuses du procédé :
l'étape e) est une étape de soudage laser d'une extrémité libre de la goupille à un bord débouchant de l'alésage commun ;
le procédé est essentiellement dépourvu d'étape de collage ; et/ou la pression contrôlée exercée sur la lame entre les deux éléments d'appui est comprise entre au moins 0,1 MPa et au plus 10 MPa.

### DESCRIPTION SOMMAIRE DES DESSINS

On va maintenant décrire un exemple de réalisation de la présente invention en référence aux dessins annexés où les mêmes références désignent d'une figure à l'autre des éléments identiques ou fonctionnellement semblables.
La Figure 1 illustre un dispositif médical de type capsule leadless dans son environnement, implanté au fond du ventricule droit d'un patient.
La Figure 2 est une vue en perspective de la partie arrière du PEH, montrant l'agencement des différents éléments permettant d'assurer l'encastrement contrôlé de la lame.
La Figure 3 est une coupe de la partie arrière du PEH, prise selon le plan de coupe A-A de la Figure 2.
La Figure 4 est une vue en élévation du bloc complet intégrant le PEH et les PCB montés dans ce bloc en même temps que le PEH.
La Figure 5 est homologue de la Figure 4, après ajout en partie arrière du bloc d'une pièce support permettant de centrer et monter l'ensemble dans le corps tubulaire de l'implant.
La Figure 6 est une vue en coupe du bloc de la Figure 5, prise selon la plan de coupe B-B de la Figure 2.
La Figure 7 est une vue en coupe du bloc de la Figure 5, prise selon la plan de coupe A-A de la Figure 2.
La Figure 8 est une vue éclatée en perspective montrant les différents éléments constitutifs du bloc de la Figure 4.
La Figure 9 est homologue de la Figure 8, après assemblage des différents éléments, dans une configuration identique à celle des Figures 5 à 7.
La Figure 10 est un organigramme explicitant les différentes étapes du procédé de réalisation du bloc PEH selon l'invention.

### DESCRIPTION DÉTAILLÉE D'UN MODE DE RÉALISATION

### PRÉFÉRENTIEL DE L'INVENTION

On va maintenant décrire un exemple de réalisation du dispositif de l'invention, dans une application à une capsule implantable autonome destinée à être implantée dans une cavité cardiaque.

Comme on l'a indiqué plus haut, cette application particulière n'est pas limitative de l'invention, dont les enseignements peuvent être appliqués à de nombreux autre types de dispositifs autonomes incorporant un récupérateur d'énergie de type PEH, que ces dispositifs soient implantables ou non, qu'il s'agisse de dispositifs médicaux ou non.

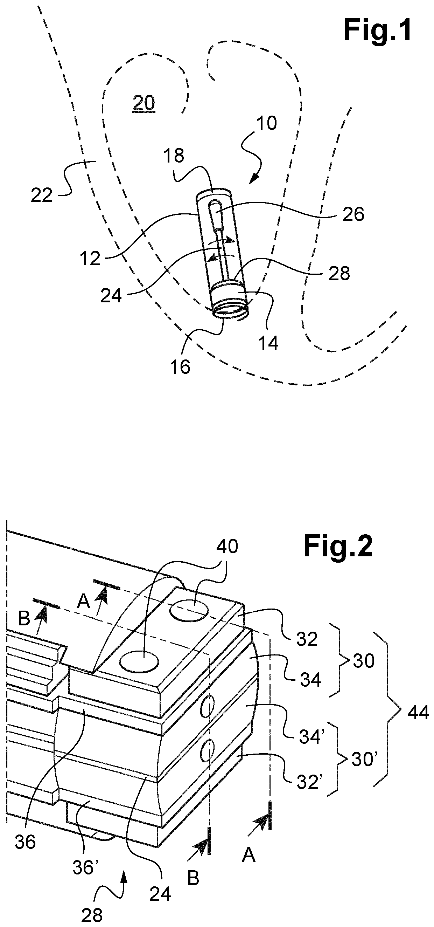

Sur la Figure 1, on a représenté un dispositif de type capsule leadless 10 dans une application à la stimulation cardiaque.

La capsule 10 se présente sous forme extérieure d'un implant avec un corps tubulaire allongé cylindrique 12 enfermant les divers circuits électroniques et d'alimentation de la capsule ainsi qu'un récupérateur d'énergie à ensemble pendulaire. Les dimensions typiques d'une telle capsule sont un diamètre de l'ordre de 6 mm pour une longueur d'environ 25 à 40 mm.

Le corps tubulaire 12 comporte à son extrémité avant (distale) 14 un élément d'ancrage saillant, par exemple une vis hélicoïdale 16 pour maintenir la capsule sur le site d'implantation. D'autres systèmes d'ancrage sont utilisables, et ne modifient en aucune façon la mise en œuvre de la présente invention. L'extrémité opposée (proximale) 18 de la capsule 10 est une extrémité libre, qui est seulement pourvue de moyens (non représentés) de liaison temporaire à un cathéter-guide ou autre accessoire d'implantation utilisé pour la mise en place ou l'explantation de la capsule, qui est ensuite désolidarisé de cette dernière.

Dans l'exemple illustré Figure 1, la capsule leadless 10 est un implant endocavitaire implanté à l'intérieur d'une cavité 20 du myocarde 22, par exemple à l'apex du ventricule droit. En variante, toujours dans une application à la stimulation cardiaque, la capsule peut être également implantée sur le septum interventriculaire ou sur une paroi auriculaire, ou encore être une capsule épicardique placée sur une, région externe du myocarde, ces différents modes d'implantation ne modifiant en aucune façon la mise en oeuvre de la présente invention. Pour assurer les fonctions de détection/stimulation, une électrode (non représentée) en contact avec le tissu cardiaque au site d'implantation assure le recueil des potentiels de dépolarisation cardiaque et/ou l'application d'impulsions de stimulation. Dans certaines formes de réalisation, la fonction de cette électrode peut être assurée par la vis d'ancrage 16, qui est alors une vis active, électriquement conductrice et reliée au circuit de détection/stimulation de la capsule.

La capsule leadless 10 est par ailleurs dotée d'un module récupérateur d'énergie dit "PEH", comprenant un ensemble pendulaire inertiel qui oscille à l'intérieur de la capsule au gré des diverses sollicitations externes auxquelles la capsule est soumise. Ces sollicitations peuvent notamment résulter : des mouvements de la paroi à laquelle est ancrée la capsule, qui sont transmis au corps tubulaire 12 par la vis d'ancrage 16 ; et/ou des variations du débit du flux sanguin dans le milieu environnant la capsule, qui produisent des oscillations du corps tubulaire 12 au rythme des battements cardiaques ; et/ou des vibrations diverses transmises par les tissus cardiaques.

L'ensemble pendulaire peut être en particulier constitué d'une lame piézoélectrique 24 encastrée en 28 à l'une de ses extrémités et dont l'extrémité opposée, libre, est couplée à une masse inertielle mobile 26. La lame piézoélectrique 24 est une lame flexible élastiquement déformable qui constitue avec la masse inertielle 26 un système pendulaire de type masse-ressort. Du fait de son inertie, la masse 26 soumet la lame 24 à une déformation de type vibratoire de part et d'autre d'une position neutre ou non déformée correspondant à une position stable de repos en l'absence de toute sollicitation.

De fait, pour ce qui est de son comportement mécanique, cet ensemble peut être assimilé à une structure de type "poutre encastrée-libre", présentant une fréquence d'oscillation propre qui est ici la fréquence sur laquelle oscille le système masse-ressort. On notera que cette fréquence d'oscillation propre, typiquement de l'ordre de quelques dizaines de hertz, est notablement supérieure à la fréquence des sollicitations cycliques externes qui correspondant à la fréquence des battements cardiaques (quelques hertz tout au plus). Ainsi, à chaque contraction cardiaque la masse inertielle (ou autre organe mécanique fonctionnellement analogue) sera sollicitée avec une amplitude plus ou moins importante, puis le système pendulaire oscillera plusieurs fois avec des amplitudes décroissantes (rebonds caractéristiques d'une oscillation périodique amortie), et enfin se stabilisera jusqu'au battement cardiaque suivant, où le cycle sollicitation/oscillations se reproduira de façon comparable.

La lame 24 assure en outre, par effet piézoélectrique, une fonction de transducteur mécanoélectrique permettant de convertir en charges électriques la contrainte mécanique de flexion qui lui est appliquée. Ces charges sont collectées par des électrodes à la surface de la lame de manière à produire un signal électrique qui, après redressement, stabilisation et filtrage, alimentera les divers circuits électroniques de la capsule.

La lame est avantageusement une lame de type bimorphe, c'est-à-dire capable de générer de l'énergie sur ses deux faces lorsqu'elle est soumise à une déformation. Ces propriétés de transduction sont typiques d'un matériau piézoélectrique tel que les céramiques PZT ou les monocristaux de type PMN-PT, titanate de baryum ou niobate de lithium. L'invention concerne plus précisément l'agencement de l'encastrement 28 qui, comme on l'a exposé en introduction, est l'un des aspects critiques de la réalisation d'un PEH performant, fiable et aisément industrialisable.

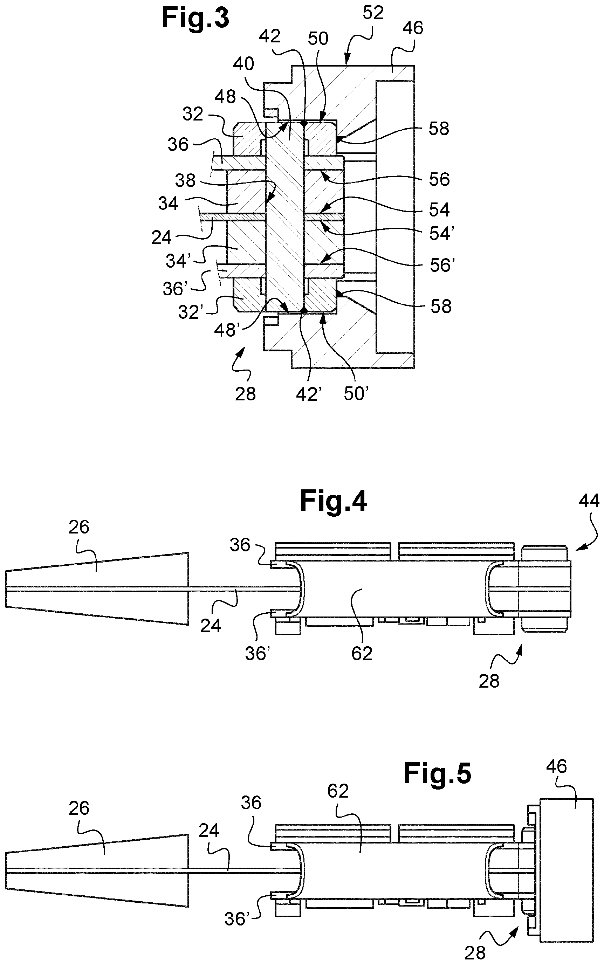

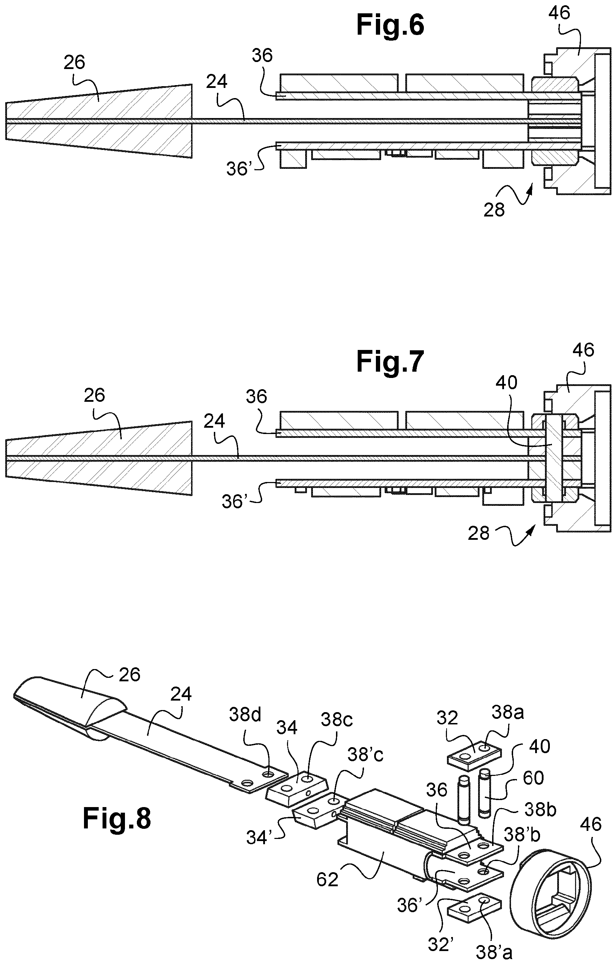

La Figure 3 et la Figure 4 illustrent, en perspective et en coupe, la structure d'un encastrement 28 réalisé selon les enseignements de l'invention. Les différentes pièces illustrées sur ces figures sont également visibles isolément sur la perspective éclatée de la Figure 8.

L'extrémité de la lame 24 située à l'opposé de la masse mobile 26 est prise en sandwich au niveau de l'encastrement 28 entre deux éléments d'appui 30, 30', de préférence symétriques, entre lesquels elle se trouve mécaniquement pincée avec une pression répartie de façon uniforme et contrôlée par les moyens de serrage que l'on décrira plus bas. Les éléments d'appui 30, 30' assurent également, du point de vue électrique, une prise de contact avec les électrodes de surface portées par la lame 24 sur l'une et l'autre de ses deux faces, et qui permettent de recueillir les charges électriques engendrées par effet piézoélectrique sous l'effet des déformations de la lame dans son mouvement d'oscillation.

Chacun des éléments d'appui 30, 30' comprend une plaque de pression respective 32, 32' et une pièce intercalaire respective 34, 34' interposée entre la plaque de pression 32, 32' et la face externe de la lame 24 située en vis- à-vis.

Dans une forme de mise en oeuvre avantageuse, une carte de circuit imprimé (ci-après PCB) 36 et/ou 36' est également interposée entre la plaque de pression respective 32, 32' et la pièce intercalaire 34, 34'. Dans la configuration illustrée sur les figures, où une lame 24 et deux PCB 36, 36' sont maintenus ensemble par les éléments d'appui, on obtient au niveau de l'encastrement 28 un empilement 44 comportant successivement :
une première plaque de pression 32,
un premier PCB 36,
une première pièce intercalaire 34,
la lame piézoélectrique 24,
une deuxième pièce intercalaire 34',
un deuxième PCB 36', et
une deuxième plaque de pression 32'.

Les pièces intercalaires 34, 34' sont avantageusement en un matériau électriquement conducteur, notamment en un métal tel qu'un acier inox, ce qui permet d'établir une liaison électrique directe entre une électrode de surface d'une première face 54 de la lame 24 et une plage conductrice correspondante 56 du PCB 36, et/ou entre une électrode de surface d'une deuxième face 54' opposée de la lame et une plage conductrice correspondante 56' du PCB 36'. On notera que le contact métal/électrode se fait avantageusement sur toute la surface de l'électrode de la lame, donc avec une impédance de contact extrêmement réduite.

L'empilement 44 est par ailleurs traversé de part en part par au moins un alésage commun 38 (par deux alésages, dans l'exemple illustré), qui est constitué par une série de trous alignés 38a, 38b, 38c, 38d, 38'a, 38'b et 38'a formés respectivement dans les éléments 32, 36, 34, 24, 34', 36' et 32' décrites ci-dessus.

Dans chacun des alésages 38 est introduite une goupille 40, dans l'exemple illustré une goupille métallique cylindrique dont la longueur est égale à l'épaisseur totale de l'empilement 44 constitué des différentes pièces que l'on vient de décrire.

À chacune de ses deux extrémités, chaque goupille 40 est solidarisée à la plaque de pression correspondante 32, 32', avantageusement par un ou plusieurs points de soudure laser tels qu'en 42, 42' (Figure 3), les plaques de pression étant à cet effet réalisées en un matériau métallique tel qu'un acier inox.

Le soudage des goupilles 40 aux plaques de pression 32, 32' a pour effet d'assujettir entre elles définitivement les différentes pièces de l'empilement 44 et de maintenir l'assemblage sous une pression constante, qui est celle qu'exerçaient les plaques de pression 32, 32' au moment de l'opération de soudage.

Un autre avantage, sur le plan électrique, est l'obtention d'une liaison directe entre les plaques de pression 32, 32', et donc entre les faces externes des PCB respectifs 36, 36' contre lesquelles appuient les faces internes de ces plaques de pression, offrant ainsi une liaison électrique supplémentaire entre les deux PCB, par exemple une liaison de masse. Pour éviter toute liaison galvanique ou courant de fuite avec les faces internes des PCB 36, 36' et/ou avec les électrodes de la lame 24, les goupilles 40 sont revêtues sur toute leur longueur, à l'exception de leurs extrémités, d'un isolant 60 (Figure 8) tel qu'une gaine thermorétractable ou un revêtement d'un matériau isolant tel que le parylène, déposé sous vide. En variante, on peut prévoir entre la goupille 40 et la surface interne de l'alésage 38 un jeu fonctionnel suffisant pour éviter ces risques électriques.

Enfin, du point de vue mécanique, on notera que la présence d'un ou plusieurs trous au centre de chacune des pièces traversées par l'alésage 38 assure une répartition plus uniforme des contraintes qu'avec une pièce qui serait dépourvue de toute discontinuité.

L'empilement 44 et les goupilles 40 soudées forment avec les PCB 36, 36' (reliés entre eux par une nappe de conducteurs flexibles 62) un sous-ensemble monobloc, qui est illustré en vue de côté à la Figure 4.

Ce sous-ensemble est monté dans un réceptacle d'une pièce support 46 de forme conjuguée, avec des faces internes 48, 48' recevant sans jeu les faces externes respectives 50, 50' des plaques de pression 32, 32' afin de recevoir et centrer précisément l'empilement dans la pièce support 46.

La pièce support 46 est avantageusement réalisée en un métal tel que le titane, ce qui permet d'y assujettir définitivement l'empilement 44 par exemple par des points de soudure laser tels qu'en 58 (Figure 3).

La face externe 52 de la pièce support 50 présente, quant à elle, une surface généralement cylindrique propre à venir se loger à l'intérieur du corps tubulaire 12 de la capsule leadless 10 au moment de l'assemblage de celle-ci.

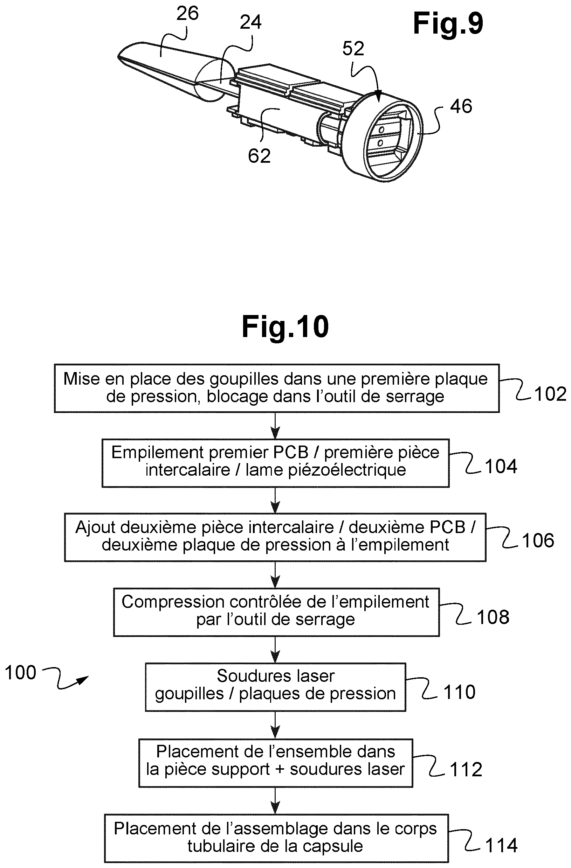

L'ensemble final ainsi obtenu est illustré : en vue de côté à la Figure 5 ; en coupe aux Figures 6 et 7 (respectivement par les plans de coupe A-A et B-B de la Figure 2) ; en perspective éclatée à la Figure 8; et en perspective non éclatée à la Figure 9.

On va maintenant décrire les différentes opérations successibles du processus d'assemblage permettant d'obtenir l'ensemble que l'on vient de décrire, en référence à l'organigramme 100 de la Figure 10.

La première étape (étape 102) consiste à mettre en place les deux goupilles 40 dans l'une des plaques de pression, par exemple la plaque de pression 32', elle-même bloquée dans un outil de serrage. L'empilement 44 est alors formé (étape 104) par ajout successif : du premier PCB 36' ; de la première pièce intercalaire 34' ; et de la lame piézoélectrique 24.

La mise en place est poursuivie (étape 106) par réitération des opérations précédentes avec ajout des derniers éléments de l'empilement : la deuxième pièce intercalaire 34 ; le deuxième PCB 36 ; enfin la deuxième plaque de pression 32.

L'outil de serrage vient ensuite (étape 108) appliquer sur les deux plaques de pression 32, 32' une pression précisément contrôlée, typiquement comprise entre 0,1 MPa et 10 MPa selon les caractéristiques propres spécifiques à la lame piézoélectrique utilisée.

Des soudures laser 42, 42' sont alors réalisées (étape 110) entre les goupilles 40 et chacune des plaques de pression 32, 32'.

Une fois ce sous-système assemblé (correspondant à celui illustré à la

Figure 4), il est positionné (étape 112) dans la pièce de support et de centrage 46, à laquelle il est fixé par des soudures laser 58 réalisées entre les plaques de pression 32, 32' et la pièce support 46. L'ensemble alors obtenu est celui illustré aux Figures 5 et 9.

Cet ensemble est enfin placé dans le corps tubulaire 12 de la capsule leadless (étape 114) par des techniques en elles-mêmes connues de mise en place, de centrage et de fixation, telles que celles décrites par exemple dans le US 2019/381325 A1 (Regnier).

## Revendications

1. Un dispositif médical actif (10) de type capsule autonome implantable, comprenant une enveloppe tubulaire allongée (12) logeant un module de récupération d'énergie, dans lequel le module de récupération d'énergie comprend un ensemble pendulaire oscillant soumis à des sollicitations externes appliquées à l'enveloppe tubulaire, l'ensemble pendulaire oscillant comprenant une lame piézoélectrique (24) élastiquement déformable, avec une extrémité encastrée (28) et une extrémité opposée libre couplée à une masse inertielle (26), la lame piézoélectrique (24) étant apte à convertir en un signal électrique oscillant l'énergie mécanique produite par les oscillations de l'ensemble pendulaire, et dans lequel l'enveloppe tubulaire loge également une monture de lame (44), la monture de lame comprenant deux éléments d'appui (30, 30') entre lesquels est prise en sandwich l'extrémité encastrée de la lame piézoélectrique (24), et un moyen de serrage mutuel des éléments d'appui, et chaque élément d'appui (30, 30') de la monture de lame (44) comprend
i) une pièce intercalaire (34, 34') dont une face interne sollicite en appui une face correspondante (54, 54') de la lame piézoélectrique (24), et
ii) une plaque de pression (32, 32') dont une face interne sollicite en appui une face externe de la pièce intercalaire (34, 34'), **caractérisé *en ce que*** les pièces intercalaires (34, 34') et les plaques de pression (32, 32') des deux éléments d'appui sont traversées de part en part par au moins un alésage commun (38a...38'd) s'étendant dans une direction transversale par rapport à un axe longitudinal de la lame piézoélectrique (24), ledit au moins un alésage commun (38a...38'd) recevant une goupille d'arrêt (40), *et **en ce que*** la monture de lame (44) comprend en outre un moyen (42, 42') de maintien en position et d'assujettissement de la goupille (40) dans ledit au moins un alésage commun (38a...38'd).

2. Le dispositif de la revendication 1, dans lequel le moyen de maintien en position et d'immobilisation de la goupille (40) comprend un soudage laser (42, 42') d'une extrémité libre de la goupille (40) à un bord débouchant de l'alésage commun (38a...38'd).

3. Le dispositif de la revendication 1, dans lequel la pièce intercalaire (34, 34') dont une face interne est en appui contre une face correspondante (54, 54') de la lame piézoélectrique (24) est en contact mécanique avec ladite face correspondante de la lame piézoélectrique'(24).

4. Le dispositif de la revendication 3, dans lequel la pièce intercalaire (34, 34') comprend sur sa face interne un matériau conducteur apte à établir en outre un contact électrique avec ladite face correspondante de la lame piézoélectrique (24), au niveau d'une électrode de surface de ladite lame piézoélectrique (24).

5. Le dispositif de la revendication 1,
comprenant en outre un circuit imprimé (36, 36') interposé entre la pièce intercalaire (34, 34') et la plaque de pression (32, 32') d'au moins l'un des éléments d'appui (30, 30') de la monture de lame (44),
et dans lequel la plaque de pression (32, 32') comprend, sur une face interne, un matériau conducteur en contact électrique avec une face externe correspondante du circuit imprimé (36, 36').

6. Le dispositif de la revendication 5, dans lequel
la pièce intercalaire (34, 34') comprend sur sa face interne un matériau conducteur apte à établir un contact électrique avec ladite face correspondante de la lame piézoélectrique (24), au niveau d'une électrode de surface de ladite lame piézoélectrique (24),
la pièce intercalaire (34, 34') comprend sur sa face externe un matériau conducteur apte à établir un contact électrique avec une face interne du circuit imprimé (36, 36'), et
les matériaux conducteurs des faces interne et externe de la pièce intercalaire (34, 34') sont électriquement couplés entre eux.

7. Le dispositif de la revendication 6, dans lequel la pièce intercalaire (34, 34') est une pièce monobloc formée d'un seul matériau conducteur.

8. Le dispositif de la revendication 1, dans lequel les pièces intercalaires (34, 34'), les plaques de pression (32, 32') et la goupille (40) sont chacune des pièces métalliques massives.

9. Le dispositif de la revendication 1, dans lequel la monture de lame (44) est essentiellement dépourvue d'élément interposé entre les pièces intercalaires (34, 34') et l'extrémité encastrée de la lame piézoélectrique (24).

10. Le dispositif de la revendication 1, dans lequel la monture de lame (44) est essentiellement dépourvue de points de collage.

11. Le dispositif de la revendication 1, dans lequel l'enveloppe tubulaire allongée loge également une pièce support (46) de forme externe tubulaire (52) conjuguée de la forme interne de l'enveloppe tubulaire (12), et comprenant à l'une de ses extrémités un réceptacle (48, 48') apte à recevoir et à centrer la monture de lame (44) et à assujettir celle-ci par ajustement sans jeu au niveau de faces externes (50, 50') des plaques de pression (32, 32').

12. Le dispositif de la revendication 1, dans lequel la goupille (40) est une pièce métallique revêtue d'un matériau électriquement isolant (60) apte à éviter une liaison galvanique et/ou un courant de fuite au sein de la lame piézoélectrique (24).

13. Un procédé de fabrication d'un dispositif selon l'une des revendications précédentes, comprenant les étapes suivantes :
a) obtention d'une lame piézoélectrique (24) ;
b) placement de deux éléments d'appui (30, 30') de part et d'autre d'une extrémité encastrée (28) de la lame piézoélectrique (24), chaque élément d'appui (30, 30') comprenant i) une pièce intercalaire (34, 34') dont une face interne sollicite en appui une face correspondante de la lame piézoélectrique (24), et ii) une plaque de pression (32, 32') dont une face interne sollicite en appui une face externe de la pièce intercalaire (34, 34'), avec le cas échéant un circuit imprimé (36, 36') interposé entre la pièce intercalaire (34, 34') et la plaque de pression (32, 32') d'au moins l'un des éléments d'appui (30, 30') de la monture de lame (44) ;
c) mise en place d'une goupille (40) dans un alésage commun (38a...38'd) traversant de part en part les pièces intercalaires (34, 34') et les plaques de pression (32, 32') des deux éléments d'appui dans une direction transversale par rapport à un axe longitudinal de la lame piézoélectrique (24) ;
d) mise sous pression contrôlée de la lame entre les deux éléments d'appui (30, 30'), jusqu'à atteindre un effort transversal prédéterminé de serrage de la lame piézoélectrique (24) entre les deux éléments d'appui (30, 30') ; et
e) tout en maintenant ledit effort transversal prédéterminé, assujettissement définitif de la goupille (40) dans ledit au moins un alésage commun (38a...38'd).

14. Le procédé de la revendication 13, dans lequel l'étape e) est une étape de soudage laser (42, 42') d'une extrémité libre de la goupille (40) à un bord débouchant de l'alésage commun (38a...38'd).

15. Le procédé de la revendication 13, dans lequel le procédé est essentiellement dépourvu d'étape de collage.

16. Le procédé de la revendication 13, dans lequel ladite pression contrôlée exercée sur la lame (24) entre les deux éléments d'appui (30, 30') est comprise entre au moins 0,1 MPa et au plus 10 MPa.

## Patentansprüche

1. Eine aktive medizinische Vorrichtung (10) vom Typ implantierbare autonome Kapsel, umfassend ein langgezogenes rohrförmiges Gehäuse (12), das ein Energierückgewinnungsmodul aufnimmt,
wobei das Energierückgewinnungsmodul eine hin- und herschwingende Pendelanordnung umfasst, die äußeren Beanspruchungen unterliegt, die auf das rohrförmige Gehäuse aufgebracht werden, wobei die hin- und herschwingende Pendelanordnung ein elastisch verformbares piezoelektrisches Plättchen (24) umfasst, mit einem eingelassenen Ende (28) und einem gegenüberliegenden freien Ende, das mit einer trägen Masse (26) gekoppelt ist, wobei das piezoelektrische Plättchen (24) imstande ist, die von den Hin- und Herschwingungen der Pendelanordnung erzeugte mechanische Energie in ein elektrisches Signal umzuwandeln,
und wobei das rohrförmige Gehäuse ferner einen Plättchenträger (44) aufnimmt, wobei der Plättchenträger zwei Lagerelemente (30, 30') umfasst, zwischen denen das eingelassene Ende (28) des piezoelektrischen Plättchens (24) sandwichartig im Eingriff ist, und ein Mittel zum gegenseitigen Festspannen der Lagerelemente umfasst, und wobei jedes Lagerelement (30, 30') des Plättchenträgers (44) umfasst:
i) ein Zwischenteil (34, 34'), von dem eine Innenfläche an eine entsprechende Fläche (54, 54') des piezoelektrischen Plättchens (24) gedrückt wird, und
ii) eine Andruckplatte (32, 32'), von der eine Innenfläche an eine Außenfläche des Zwischenteils (34, 34') gedrückt wird,
***dadurch gekennzeichnet, dass*** die Zwischenteile (34, 34') und die Andruckplatten (32, 32') der zwei Lagerelemente (30, 30') von einer Seite zur anderen von wenigstens einer gemeinsamen Bohrung (38a...38'd) durchquert sind, die sich in einer Querrichtung zu einer Längsachse des piezoelektrischen Plättchens (24) erstreckt, wobei die wenigstens eine gemeinsame Bohrung (38a...38'd) einen Arretierstift (40) aufnimmt,
*und dass* der Plättchenträger (44) ferner ein Mittel (42, 42') zum Festhalten in Position und Festlegen des Stiftes (40) in der wenigstens einen gemeinsamen Bohrung (38a...38'd) umfasst.

2. Die Vorrichtung nach Anspruch 1, wobei das Mittel zum Festhalten in Position und Festlegen des Stiftes (40) eine Laserverschweißung (42, 42') eines freien Endes des Stiftes (40) an einem Mündungsrand der gemeinsamen Bohrung (38a...38'd) umfasst.

3. Die Vorrichtung nach Anspruch 1, wobei das Zwischenteil (34, 34'), von dem eine Innenfläche in Anlage gegen eine entsprechende Fläche (54, 54') des piezoelektrischen Plättchens (24) ist, in mechanischem Kontakt mit der entsprechenden Fläche des piezoelektrischen Plättchens (24) ist.

4. Die Vorrichtung nach Anspruch 3, wobei das Zwischenteil (34, 34') auf seiner Innenfläche ein leitendes Material umfasst, das imstande ist, ferner einen elektrischen Kontakt mit der entsprechenden Fläche des piezoelektrischen Plättchens (24), im Bereich einer Oberflächenelektrode des piezoelektrischen Plättchens (24), herzustellen.

5. Die Vorrichtung nach Anspruch 1, ferner umfassend eine gedruckte Schaltung (36, 36'), die zwischen dem Zwischenteil (34, 34') und der Andruckplatte (32, 32') des wenigstens einen der Lagerelemente (30, 30') des Plättchenträgers (44) angeordnet ist, und wobei die Andruckplatte (32, 32'), auf einer Innenfläche, ein leitendes Material in elektrischem Kontakt mit einer entsprechenden Außenfläche der gedruckten Schaltung (36, 36') umfasst.

6. Die Vorrichtung nach Anspruch 5, wobei das Zwischenteil (34, 34') auf seiner Innenfläche ein leitendes Material umfasst, das imstande ist, einen elektrischen Kontakt mit der entsprechenden Fläche des piezoelektrischen Plättchens (24), im Bereich einer Oberflächenelektrode des piezoelektrischen Plättchens (24), herzustellen, das Zwischenteil (34, 34') auf seiner Außenfläche ein leitendes Material umfasst, das imstande ist, einen elektrischen Kontakt mit einer Innenfläche der gedruckten Schaltung (36, 36') herzustellen, und
die leitenden Materialien der Innen- und Außenfläche des Zwischenteils (34, 34') elektrisch miteinander gekoppelt sind.

7. Die Vorrichtung nach Anspruch 6, wobei das Zwischenteil (34, 34') ein einteiliges Teil ist, das aus einem einzigen leitenden Material gebildet ist.

8. Die Vorrichtung nach Anspruch 1, wobei die Zwischenteile (34, 34'), die Andruckplatten (32, 32') und der Stift (40) jeweils massive Metallteile sind.

9. Die Vorrichtung nach Anspruch 1, wobei der Plättchenträger (44) im Wesentlichen kein Element aufweist, das zwischen den Zwischenteilen (34, 34') und dem eingelassenen Ende des piezoelektrischen Plättchens (24) angeordnet ist.

10. Die Vorrichtung nach Anspruch 1, wobei der Plättchenträger (44) im Wesentlichen keine Klebepunkte aufweist.

11. Die Vorrichtung nach Anspruch 1, wobei das rohrförmige Gehäuse ferner ein Trägerteil (46) aufnimmt, das eine rohrförmige äußere Form (52), die an die innere Form des rohrförmigen Gehäuse (12) angepasst ist, aufweist und an einem seiner Enden ein Behältnis (48, 48') umfasst, das imstande ist, den Plättchenträger (44) aufzunehmen und zu zentrieren und diesen durch spielfreies Einpassen im Bereich von Außenflächen (50, 50') der Andruckplatten (32, 32') festzulegen.

12. Die Vorrichtung nach Anspruch 1, wobei der Stift (40) ein metallisches Teil ist, das mit einem elektrisch isolierenden Material (60) beschichtet ist, das imstande ist, eine galvanische Verbindung und/oder einen Kriechstrom im Inneren des piezoelektrischen Plättchens (24) zu verhindern.

13. Ein Verfahren zum Herstellen einer Vorrichtung nach einem der vorhergehenden Ansprüche, umfassend die folgenden Schritte:
a) Erhalten eines piezoelektrischen Plättchens (24);
b) Platzieren von zwei Lagerelementen (30, 30') auf beiden Seiten eines eingelassenen Endes (28) des piezoelektrischen Plättchens (24), wobei jedes Lagerelement (30, 30') umfasst: i) ein Zwischenteil (34, 34'), von dem eine Innenfläche an eine entsprechende Fläche des piezoelektrischen Plättchens (24) gedrückt wird, und ii) eine Andruckplatte (32, 32'), von der eine Innenfläche an eine äußere Fläche des Zwischenteils (34, 34') gedrückt wird, mit gegebenenfalls einer gedruckten Schaltung (36, 36'), die zwischen dem Zwischenteil (34, 34') und der Andruckplatte (32, 32') des wenigstens einen der Lagerelemente (30, 30') des Plättchenträgers (44) angeordnet ist;
c) Einsetzen eines Stiftes (40) in einer gemeinsamen Bohrung (38a...38'd), die von einer Seite zur anderen die Zwischenteile (34, 34') und die Andruckplatten (32, 32') der zwei Lagerelemente in einer Querrichtung zu einer Längsachse des piezoelektrischen Plättchens (24) durchquert;
d) kontrolliertes Druckbeaufschlagen des Plättchens zwischen den zwei Lagerelementen (30, 30'), bis eine vorab bestimmte quergerichtete Kraft zum Festspannen des piezoelektrischen Plättchens (24) zwischen den zwei Lagerelementen (30, 30') erreicht wird; und
e) unter Aufrechterhaltung der vorab bestimmten quergerichteten Kraft, endgültiges Festlegen des Stiftes (40) in der wenigstens einen gemeinsamen Bohrung (38a...38'd).

14. Das Verfahren nach Anspruch 13, bei dem Schritt e) ein Schritt des Laserverschweißens (42, 42') eines freien Endes des Stiftes (40) an einem Mündungsrand der gemeinsamen Bohrung (38a...38'd) ist.

15. Das Verfahren nach Anspruch 13, bei dem das Verfahren im Wesentlichen keinen Schritt des Klebens aufweist.

16. Das Verfahren nach Anspruch 13, bei dem der kontrollierte Druck, der auf das Plättchen (24) zwischen den zwei Lagerelementen (30, 30') ausgeübt wird, zwischen wenigstens 0,1 MPa und höchstens 10 MPa beträgt.

## Claims

1. An active medical device (10) of an autonomous implantable capsule type, comprising an elongated tubular envelope (12) housing an energy harvesting module,
wherein the energy harvesting module comprises an oscillating pendular unit subjected to external stresses applied to the tubular envelope, the oscillating pendular unit comprising an elastically deformable piezoelectric beam (24) having a clamped end (28) and an opposite, free end coupled to an inertial mass (26), the piezoelectric beam (24) being adapted to convert into an oscillating electric signal a mechanical energy produced by oscillations of the pendular unit,
and wherein the tubular envelope also houses a beam frame (44), the beam frame comprising two pressing elements (30, 30') between which the clamped end of the piezoelectric beam (24) is taken in sandwich, and a means for tightening the pressing elements together, and each pressing element (30, 30') of the beam frame (44) comprises i) an intermediate part (34, 34'), an internal face of which presses on a corresponding face (54, 54') of the piezoelectric beam (24), and ii) a pressure plate (32, 32'), an internal face of which presses on an external face of the intermediate part (34, 34'), ***characterized in that***
the intermediate parts (34, 34') and the pressure plates (32, 32') of the two pressing elements are passed through by at least one common bore (38a...38'd) extending in a transverse direction with respect to a longitudinal axis of the piezoelectric beam (24), said at least one common bore (38a...38'd) receiving a locking pin (40), ***and in that***
the beam frame (44) further comprises a means (42, 42') for holding and fastening the pin (40) in position into said at least one common bore (38a...38'd).

2. The device of claim 1, wherein the means for holding and immobilizing the pin (40) in position comprises a laser welding (42, 42') of a free end of the pin (40) at an open edge of the common bore (38a...38'd).

3. The device of claim 1, wherein the intermediate part (34, 34'), an internal face of which presses against a corresponding face (54, 54') of the piezoelectric beam (24), is in mechanical contact with said corresponding face of the piezoelectric beam (24).

4. The device of claim 3, wherein the intermediate part (34, 34') comprises on the internal face thereof a conductive material capable of further establishing an electrical contact with said corresponding face of the piezoelectric beam (24), at a surface electrode of said piezoelectric beam (24).

5. The device of claim 1,
further comprising a printed circuit board (36, 36') interposed between the intermediate part (34, 34') and the pressure plate (32, 32') of at least one of the pressing elements (30, 30') of the beam frame (44),
and wherein the pressure plate(32, 32') comprises, on an internal face, a conductive material in electrical contact with the corresponding external face of the printed circuit board (36, 36').

6. The device of claim 5, wherein
the intermediate part (34, 34') comprises, on its internal face, a conductive material capable of establishing an electrical contact with said corresponding face of the piezoelectric beam (24), at a surface electrode of said piezoelectric beam (24),
the intermediate part (34, 34') comprises, on its external face, a conductive material capable of establishing an electrical contact with an internal face of the printed circuit board (36, 36'), and
the conductive materials of the internal and external faces of the intermediate part (34, 34') are electrically coupled to each other.

7. The device of claim 6, wherein the intermediate part (34, 34') is a single-piece part formed of a single conductive material.

8. The device of claim 1, wherein the intermediate parts (34, 34'), the pressure plates (32, 32') and the pin (40) are each massive metal parts.

9. The device of claim 1, wherein the beam frame (44) is essentially devoid of element interposed between the intermediate parts (34, 34') and the clamped end of the piezoelectric beam (24).

10. The device of claim 1, wherein the beam frame (44) is essentially devoid of bonding points.

11. The device of claim 1, wherein the elongated tubular envelope also houses a support part (46) having an external tubular shape (52) mating an internal shape of the tubular envelope (12), and comprising at one of its ends a receptacle (48, 48') capable of receiving and centring the beam frame (44) and of fastening the latter by press-fit at external faces (50, 50') of the pressure plates (32, 32').

12. The device of claim 1, wherein the pin (40) is a metal part coated with an electrically insulating material (60) adapted to avoid a galvanic connection and/or a leak current within the piezoelectric beam (24).

13. A method of manufacturing a device according to claim 1, comprising the following steps:
a) obtaining a piezoelectric beam (24);
b) placing two pressing elements (30, 30') on either side of a clamped end (28) of the piezoelectric beam (24), each pressing element (30, 30') comprising i) an intermediate part (34, 34'), an internal face of which presses on a corresponding face of the piezoelectric beam (24), and ii) a pressure plate (32, 32'), an internal face of which presses on an external face of the intermediate part (34, 34'), optionally with a printed circuit board (36, 36') interposed between the intermediate part (34, 34') and the pressure plate (32, 32') of at least one of the pressing elements (30, 30') of the beam frame (44);
c) positioning a pin (40) into a common bore (38a...38'd) passing through the intermediate parts (34, 34') and the pressure plates (32, 32') of the two pressing elements in a transverse direction with respect to a longitudinal axis of the piezoelectric beam (24);
d) pressing the beam under controlled pressure between the two pressing elements (30, 30'), until reaching a predetermined transverse force of clamping of the piezoelectric beam (24) between the two pressing elements (30, 30'); and
e) while maintaining said predetermined transverse force, definitively fastening the pin (40) into said at least one common bore (38a...38'd).

14. The method of claim 13, wherein step e) is a step of laser welding (42, 2') of a free end of the pin(40) to an open edge of the common bore (38a...38'd).

15. The method of claim 13, wherein the method is essentially devoid of bonding step.

16. The method of claim 13, wherein said controlled pressure exerted on the beam (24) between the two pressing elements (30, 30') is comprised between at least 0.1 MPa and at most 10 MPa.
